(19) Europäisches Patentamt
European Patent Office
Office européen des brevets

(11) **EP 4 335 376 B1**

(12) **EUROPEAN PATENT SPECIFICATION**

(45) Date of publication and mention
of the grant of the patent:
**26.02.2025 Bulletin 2025/09**

(21) Application number: **23195877.8**

(22) Date of filing: **07.09.2023**

(51) International Patent Classification (IPC):
**A61B 6/03** (2006.01)　　　**A61B 6/06** (2006.01)

(52) Cooperative Patent Classification (CPC):
**A61B 6/06; A61B 6/032; A61B 6/5258; G21K 1/04;
H05G 1/52**

(54) **TUNING METHOD FOR COLLIMATOR, CONTROLLER, COLLIMATOR AND CT MACHINE**

EINSTELLUNGSVERFAHREN FÜR KOLLIMATOR, STEUERUNG, KOLLIMATOR UND
CT-MASCHINE

PROCÉDÉ DE RÉGLAGE POUR COLLIMATEUR, CONTRÔLEUR, COLLIMATEUR ET MACHINE
DE TOMODENSITOMÉTRIE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **09.09.2022　CN 202211104140**

(43) Date of publication of application:
**13.03.2024 Bulletin 2024/11**

(73) Proprietor: **Siemens Healthineers AG
91301 Forchheim (DE)**

(72) Inventors:
• **ZHU, Jia Peng**
**Shanghai, 201318 (CN)**
• **ZHANG, Li Dong**
**Shanghai, 201316 (CN)**
• **ZHANG, Jian**
**Shanghai, 201318 (CN)**
• **ZHAO, Ji Feng**
**Shanghai, 200120 (CN)**
• **YU, Feng Hai**
**Shanghai, 201201 (CN)**

(74) Representative: **Horn Kleimann Waitzhofer
Schmid-Dreyer
Patent- und Rechtsanwälte PartG mbB
Theresienhöhe 12
80339 München (DE)**

(56) References cited:
**WO-A1-2011/068145　　US-A- 4 991 189
US-B2- 8 331 527**

**Description**

TECHNICAL FIELD

The present invention relates to a tuning method for a collimator of a CT machine, in particular to a tuning method for a collimator of a CT machine, a controller for a collimator, a collimator, and a CT machine.

BACKGROUND ART

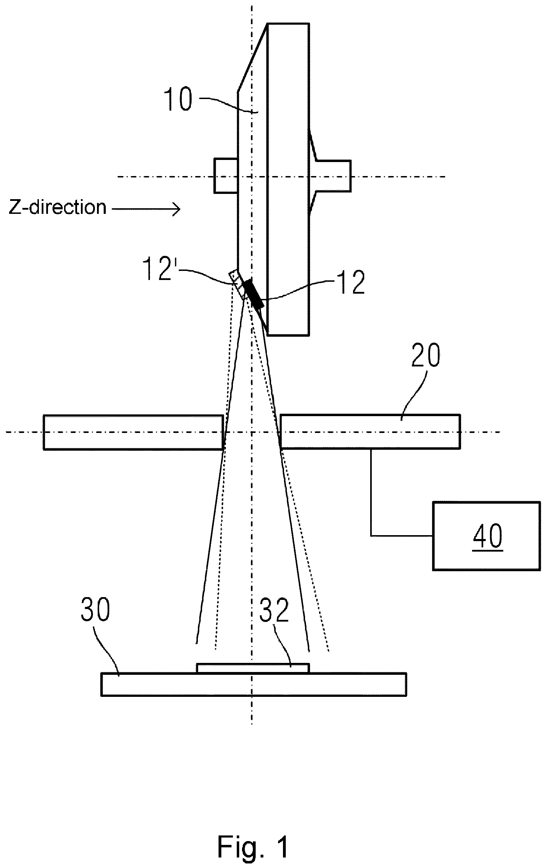

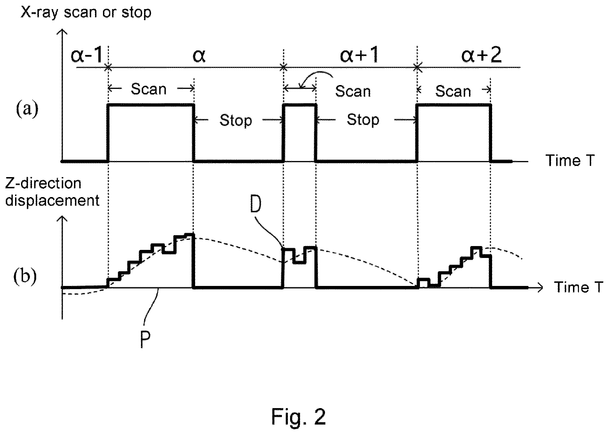

In computed tomography (CT), instability of focal positions of an X-ray tube may lead to a decrease in image quality and even result in image artifacts. Focus shifts are mainly caused by the thermal conditions of an X-ray tube.
It is well known that during a scan, the anode plate of the X-ray tube expands when heated, and the focal position moves in one direction along the Z-axis; when the scan has stopped, as the temperature decreases, the anode plate of the X-ray tube will be turned around and move in the opposite direction. This phenomenon is particularly evident in the Z-direction of the tube, and if no corrective measures are taken, an X-ray having passed through the collimator (TCO) will deviate from an effective detector area, which will result in a decrease in image quality. As shown in Fig. 1, the CT machine comprises an anode plate 10 for generating X-rays. The collimator used for the CT machine comprises a slot plate 20 and a controller 40. When the anode plate expands due to heat, the focal position 12 will move in one direction along the Z-axis to 12', so when the scan target 32 on the scanning table 30 is scanned, the X-ray will have displacements.
US 8,331,527 B2 discloses an X-ray tube that is heated under an X-ray scan condition and thus a focal point is shifted. Artifact occurs in a reconstructed image due to the focal point shift. The invention has an object to enable correction of the focal point shift caused by heating the tube. The applicant and inventor of this application has confirmed that the focal point shift amount of the tube varies in accordance with whether it is in a heating direction or cooling direction when viewed from a past sequence. Therefore, the sequence record and the focal point shift amount based on the heating and cooling directions are stored as data in a storage device. A just near past sequence record when viewed from now is stored in a storage unit, and the tube temperature is detected by a tube temperature detector. On the basis of this temperature and the data in the storage unit, heating or cooling and the present accumulated heat capacity are determined in a determining unit, and the storage device is accessed to determine a focal point shift amount. The position of the tube is corrected on the basis of this shift amount.
To address this problem, thermal Z-control is the most commonly used to compensate for focus shift. With this method, real-time data obtained by the detector are used, and the distance to be covered by the slot plate is calculated to ensure that the X-ray remains within an effective area of the scan target 32.
As shown in Fig. 2, the Z-direction displacement of segment P represents the Z-direction displacement of the slot plate during a scan, and since no X-ray is emitted during the scan stop period from the closing of the previous scan till the start of the next scan, thermal Z-control cannot be performed. Therefore, when a scan has stopped, the Z-direction displacement of the slot plate is within the scan stop period, and the slot plate has no displacement. When the next scan starts, the actual focus position has substantially deviated from the slot plate position, which results in a decrease in image quality at the beginning of the scan.

SUMMARY OF THE INVENTION

An objective of the present invention is to provide a tuning method for a collimator of a CT machine, which makes it possible to control the displacement of the slot plate during the scan stop stage, thereby improving the image scan quality of the CT machine.
The present invention provides a collimator for a CT machine. The CT machine comprises an anode plate. The collimator comprises a slot plate and a controller. The tuning method for a collimator of a CT machine, which, in each cycle of an X-ray scan operation and a stop, comprises: a detection step of determining whether an X-ray is generated from the anode plate; if an X-ray is detected in the detection step, the controller calculates the Z-direction heating displacement $\Delta z_{Thermal}^{(\alpha)}$ of the slot plate during the current scan, wherein $\alpha$ indicates the current cycle; the controller drives the slot plate to move by the heating displacement in the Z-direction; if no X-ray is detected in the detection step, the controller calculates the total Z-direction heating displacement $z_{Thermal}^{(\alpha)}$ of the slot plate at the end of the current scan; the controller, on the basis of the stop time of the X-ray, calculates the cooling displacement $z_{Thermal}^{(\alpha)\,\prime}$ of the slot plate during the current stop; and the controller drives the slot plate to move.
With the tuning method for a collimator of a CT machine provided by the present invention, by "learning control", the slot plate can, on the basis of the total displacement of the previous scan and the stop time, control the displacement of the slot plate during the scan stop stage, thereby minimizing the substantial jitter of the slot plate at the beginning and at the end of a scan, which, in turn, reduces risks of image quality degradation.

In an illustrated embodiment of the tuning method for a collimator of a CT machine, the cooling displacement $z_{Thermal}^{(\alpha)\,\prime}$ at the time of the current stop is the difference between the total heating displacement $z_{Thermal}^{(\alpha)}$ of the current scan and $f(\Delta T_\alpha)$, a function of the current stop time $\Delta T\alpha$, namely $z_{Thermal}^{(\alpha)\,\prime} = z_{Thermal}^{(\alpha)} - f(\Delta T_\alpha)$. Thus, a linear model may be used to estimate the cooling displacement generated at the time when a scan is performed again after cooling following a scan stop.

In another schematic embodiment of the tuning method for a collimator of a CT machine, $f(\Delta T_\alpha)$, a function of the current stop time, is a linear function, namely $f(\Delta T_\alpha) = \kappa \cdot \Delta T_\alpha$, wherein $\kappa$ is the thermal coefficient of the anode plate. During the scan stop $\Delta T$, the X-ray tube cools down because no scan is performed. Therefore, the focal position keeps drifting in a direction opposite to a heating direction. Therefore, a linear equation in units of time $\Delta T$ is established to estimate the required slot plate position before the next scan.

In another illustrated embodiment of the tuning method for a collimator of a CT machine, the method, in each cycle of an X-ray scan operation and a stop, further comprises a step of comparing the cooling displacement $z_{Thermal}^{(\alpha)\,\prime}$ at the current stop with a predetermined cooling position $Z_{min}$ of the slot plate; if the cooling displacement $z_{Thermal}^{(\alpha)\,\prime}$ is not smaller than the predetermined cooling position $Z_{min}$, the controller drives the slot plate to move by the cooling displacement $z_{Thermal}^{(\alpha)\,\prime}$; if the cooling displacement $z_{Thermal}^{(\alpha)\,\prime}$ is smaller than the predetermined cooling position $Z_{min}$, the controller drives the slot plate to move by the predetermined cooling position $Z_{min}$. When the calculated slot plate displacement exceeds a predetermined value, the displacement is a linear change, and when the calculated slot plate displacement is smaller than a predetermined value, the slot plate is displaced by the predetermined value.

In another illustrated embodiment of the tuning method for a collimator of a CT machine, the total heating displacement $z_{Thermal}^{(\alpha)}$ of the current scan is the sum of the total cooling displacement $z_{\text{Thermal}}^{(\alpha-1)\,\prime}$ of the previous scan and the heating displacement $\Delta z_{Thermal}^{(\alpha)}$ of the current scan, namely $z_{\text{Thermal}}^{(\alpha)} = z_{\text{Thermal}}^{(\alpha-1)\,\prime} + \Delta z_{\text{Thermal}}^{(\alpha)}$.

In another illustrated embodiment of the tuning method for a collimator of a CT machine, the heating displacement $\Delta z_{Thermal}^{(\alpha)}$ of the current scan is the sum of the displacements in the successive movements made by the slot plate during the current scan, namely $\Delta z_{Thermal}^{(\alpha)} = \sum_{i=1}^{i=n} \Delta z_i^{\,(\alpha)}$, wherein n is the total number of slot plate movements during the current scan. Therefore, $\Delta z_{Thermal}^{(\alpha)}$ may also be expressed as

$$z_{Thermal}^{(\alpha)} = z_{Thermal}^{(\alpha-1)} + \Delta z_{Thermal}^{(\alpha)} = z_{Thermal}^{(\alpha-1)} + \Delta z_1^{\,(\alpha)} + \Delta z_2^{\,(\alpha)} + \cdots + \Delta z_n^{(\alpha)} =$$

$z_{Thermal}^{(\alpha-1)} + \sum_{i=1}^{i=n} \Delta z_i^{\,(\alpha)}$. Therefore, the heating displacement during a scan may change by a gradient.

In another illustrated embodiment of the tuning method for a collimator of a CT machine, $f(\Delta T_\alpha)$, a function of the current stop time, is a linear function, namely $f(\Delta T_\alpha)$, $= \kappa \cdot \Delta T_\alpha$, wherein $\kappa$ is the thermal coefficient of the anode plate. Therefore, the heating displacement during a scan may change linearly.

In another illustrated embodiment of the tuning method for a collimator of a CT machine, the method, in each cycle of an X-ray scan operation and a stop, further comprises a step of comparing the calculated heating displacement $z_{Thermal}^{(\alpha)}$ of the current scanning with a predetermined heating displacement $Z_{max}$ of the slot plate; if the calculated heating displacement $z_{Thermal}^{(\alpha)}$ is not greater than the predetermined heating displacement $Z_{max}$, the controller drives the slot plate to move by the heating displacement $z_{Thermal}^{(\alpha)}$; if the calculated heating displacement $z_{Thermal}^{(\alpha)}$ is greater than the predetermined heating displacement $Z_{max}$, the controller drives the slot plate to move by the predetermined heating displacement $Z_{max}$. During a scan, the anode plate is heated, wherein, when the calculated slot plate displacement does not exceed a predetermined value, the displacement changes linearly, and when the calculated slot plate displacement is greater than a predetermined value, the slot plate is displaced by the predetermined value.

The present invention further provides a controller for a collimator of a CT machine, the controller comprising a control module for executing the above steps.

The present invention provides a collimator of a CT machine, the collimator comprising a slot plate and a controller as described above.

The present invention further provides a CT machine, which comprises a collimator as described above.

A controller, a collimator, and a CT machine provided by the present invention make it possible to, on the basis of the total displacement and stop time of the previous scan, control the displacement of the slot plate during the scan stop stage or simultaneously control the displacement of the slot plate during the scan stage, so that possibility of the slot plate undergoing any substantial jitter when the CT machine performs a scan or stops is minimized, thereby keeping the image quality stable.

BRIEF DESCRIPTION OF THE DRAWINGS

The present invention is explained in detail below in conjunction with the accompanying drawings and particular embodiments.

Fig. 1 is a schematic diagram of the Z-direction displacements of the anode plate of a collimator for a CT machine in the prior art.

Fig. 2 shows the changes in the Z-direction displacement over time according to a method for compensating for the focus shift in the prior art.

Fig. 3 shows an illustrated embodiment of the tuning method for a collimator of a CT machine according to the present invention.

Fig. 4 shows an illustrated embodiment of the tuning method for a collimator of a CT machine according to the present invention.

Fig. 5 show the changes in the Z-direction displacement over time according to the tuning method for a collimator of a CT machine shown in Fig. 3.

Fig. 6 show the changes in the Z-direction displacement over time according to the tuning method for a collimator of a CT machine shown in Fig. 4.

SPECIFIC EMBODIMENTS

To enable clearer understanding of the technical features, objectives and effects of the invention, particular embodiments of the present invention are now explained with reference to the accompanying drawings, in which identical labels indicate structurally identical components or components with similar structures but identical functions.

As used herein, "schematic" means "serving as an instance, example or illustration". No drawing or embodiment described herein as "schematic" should be interpreted as being a more preferred or more advantageous technical solution.

The various numbers used herein, instead of being strict mathematical and/or geometric limitations, also contain errors that are comprehensible to those skilled in the art and are allowable in manufacture or use.

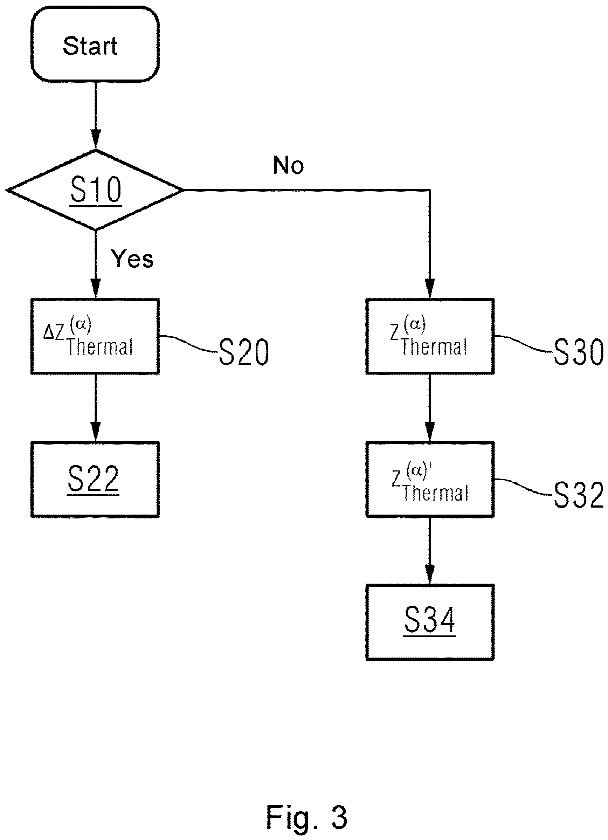

Fig. 3 is an illustrated embodiment of the tuning method for a collimator of a CT machine. As shown in Fig. 3, the tuning method for a collimator of a CT machine, in each cycle of an X-ray scan operation and a stop, comprises the steps of:

S10: a detection step of determining whether an X-ray is generated from the anode plate;

S20: if an X-ray is detected in the detection step, the controller calculates the Z-direction heating displacement $\Delta z_{Thermal}^{(\alpha)}$ of the slot plate during the current scan, wherein a indicates the current cycle;

S22: the controller drives the slot plate to move by the heating displacement in the Z-direction;

S30: if no X-rays are detected in the detection step (S10), the controller calculates the total Z-direction heating

displacement $z_{Thermal}^{(\alpha)}$ of the slot plate at the end of the current scan;

S32: the controller, on the basis of the stop time of the X-ray, calculates the cooling displacement $z_{Thermal}^{(\alpha)\,\prime}$ of the slot plate during the current stop; and

S34: the controller drives the slot plate to move.

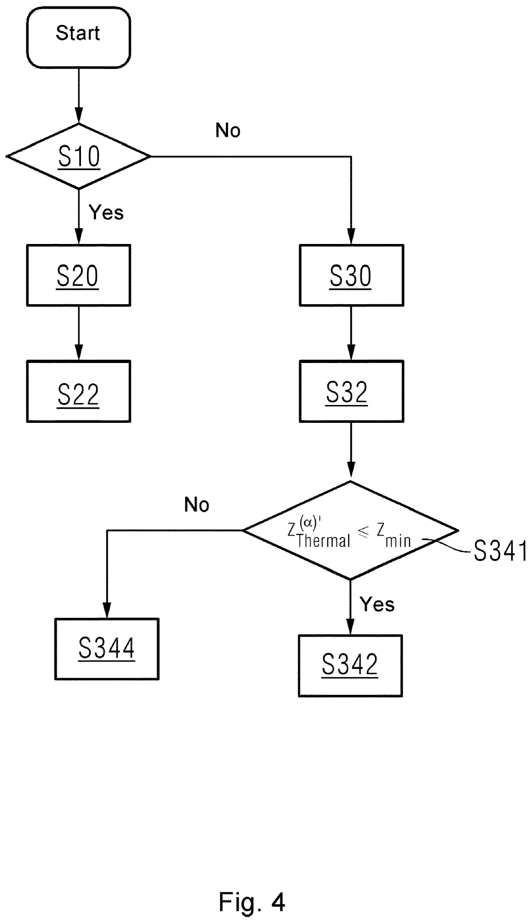

Fig. 4 is an illustrated embodiment of the tuning method for a collimator of a CT machine. As shown in Fig. 4, the tuning method for a collimator of a CT machine, in each cycle of an X-ray scan operation and a stop, comprises the steps of:

S10: a detection step of determining whether an X-ray is generated from the anode plate;

S20: if an X-ray is detected in the detection step, the controller calculates the Z-direction heating displacement $\Delta z_{Thermal}^{(\alpha)}$ of the slot plate during the current scan, wherein a indicates the current cycle;

S22: the controller drives the slot plate to move by the heating displacement in the Z-direction;

S30: if no X-ray is detected in the detection step, the controller calculates the total Z-direction heating displacement $z_{Thermal}^{(\alpha)}$ of the slot plate at the end of the current scan; the total heating displacement $z_{Thermal}^{(\alpha)}$ of the current scan is the sum of the total cooling displacement $z_{Thermal}^{(\alpha-1)\,\prime}$ of the previous scan and the heating displacement $\Delta z_{Thermal}^{(\alpha)}$ of the current scan, namely $z_{Thermal}^{(\alpha)} = z_{Thermal}^{(\alpha-1)\,\prime} + \Delta z_{Thermal}^{(\alpha)}$, and the heating displacement $\Delta z_{Thermal}^{(\alpha)}$ of the current scan is the sum of the displacements in the successive movements made by the slot plate during the current scan, namely $\Delta z_{Thermal}^{(\alpha)} = \sum_{i=1}^{i=n} \Delta z_i^{(\alpha)}$ , wherein n is the total number of slot plate movements during the current scan;

S32: the controller, on the basis of the stop time of the X-ray, calculates the cooling displacement $z_{Thermal}^{(\alpha)\,\prime}$ of the slot plate during the current stop, wherein the cooling displacement $z_{Thermal}^{(\alpha)\,\prime}$ at the time of the current stop is the difference between the total heating displacement $z_{Thermal}^{(\alpha)}$ of the current scan and $f\,(\Delta T_\alpha)$, , a function of the current stop time $\Delta T\alpha$, namely $z_{Thermal}^{(\alpha)\,\prime} = z_{Thermal}^{(\alpha)} - f\;(\Delta T_\alpha)$ , and $f(\Delta T_\alpha)$, a function of the current stop time, is a linear function, namely $f\,(\Delta T_\alpha) = \kappa \cdot \Delta T_\alpha$, wherein $\kappa$ is the thermal coefficient of the anode plate;

S341: compare the cooling displacement $z_{Thermal}^{(\alpha)\,\prime}$ at the current stop with a predetermined cooling position $Z_{min}$ of the slot plate;

S342: if the cooling displacement $z_{Thermal}^{(\alpha)\,\prime}$ is not smaller than the predetermined cooling position $Z_{min}$, the controller drives the slot plate to move by the cooling displacement $z_{Thermal}^{(\alpha)\,\prime}$ ;

S344: if the cooling displacement $z_{Thermal}^{(\alpha)\,\prime}$ is smaller than the predetermined cooling position $Z_{min}$, the controller drives the slot plate to move by the predetermined cooling position $Z_{min}$.

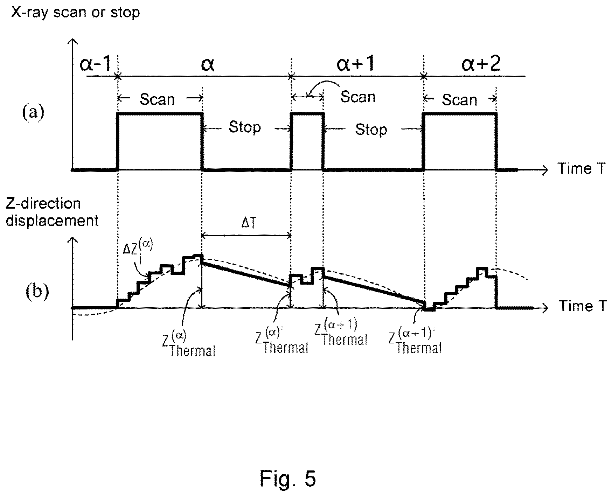

Fig. 5 show the changes in the Z-direction displacement over time according to the tuning method for a collimator of a CT machine shown in Fig. 3. As shown in Fig. 5, part (a) shows the time of an X-ray scan or a stop. Part (b) is a graph of the changes in the Z-direction displacement over time. As shown in Fig. 5, during a scan stop, the controller, on the basis of the X-ray stop time, calculates the cooling displacement $z_{Thermal}^{(\alpha)\prime}$ of the slot plate during the current stop, which is a linear displacement during the stop period $\Delta T$.

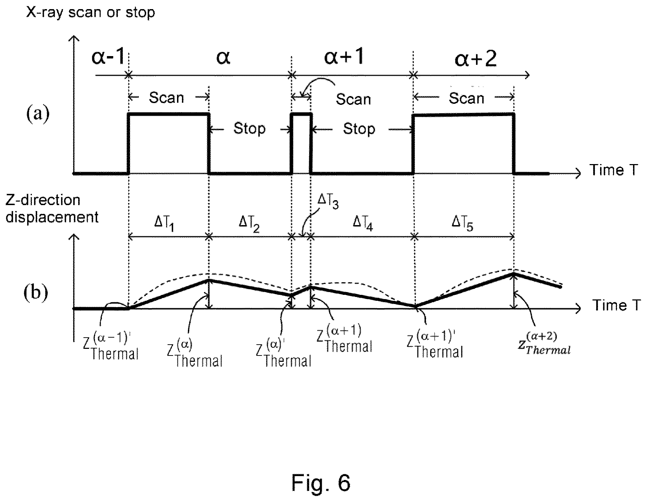

Fig. 6 show the changes in the Z-direction displacement over time according to the tuning method for a collimator of a CT machine shown in Fig. 4. In step S30, the heating displacement $z_{Thermal}^{(\alpha)}$ of the current scan may be calculated by the controller on the basis of the thermal coefficient $\kappa_{H\alpha}$ of the current scan and the time $\Delta T_{H\alpha}$ of the current scan:

$$\Delta z_{Thermal}^{(\alpha)} = \kappa_{H\alpha} \cdot \Delta T_{H\alpha}$$, wherein $\kappa_{H\alpha}$ is determined by a lookup table stored in the controller. Fig. 6 shows the changes in the Z-direction displacement over time according to the tuning method of this embodiment, wherein part (a) shows the time of an X-ray scan or a stop. Part (b) is a graph of the changes in the Z-direction displacement over time. As shown in Fig. 5, during a scan, the heating displacement $z_{Thermal}^{(\alpha)}$ is a linear displacement. During a scan stop, the controller, on the basis of the X-ray stop time, calculates the cooling displacement $z_{Thermal}^{(\alpha)\prime}$ of the slot plate at the time of the current stop, which is a linear displacement during the stop period $\Delta T$.

The present invention further provides a controller for a collimator for a CT machine, the controller comprising a control module for executing the above steps.

The present invention provides a collimator for a CT machine, which comprises a slot plate for a collimator and a controller as described above.

The present invention further provides a CT machine, which comprises a collimator as described above.

A controller, a collimator, and a CT machine provided by the present invention make it possible to, on the basis of the total displacement and stop time of the previous scan, control the displacement of the slot plate during the scan stop stage or simultaneously control the displacement of the slot plate during the scan stage, so that possibility of the slot plate undergoing any substantial jitter when the CT machine performs a scan or stops is minimized, thereby keeping the image quality stable.

It should be understood that although the description herein is based on various embodiments, it is by no means the case that each embodiment contains just one independent technical solution. Such a method of presentation is adopted herein purely for the sake of clarity. Those skilled in the art should consider the description in its entirety. The technical solutions in the various embodiments could also be suitably combined to form other embodiments understandable to those skilled in the art.

The series of detailed explanations set out above are merely particular explanations of feasible embodiments of the present invention, which are not intended to limit the scope of protection thereof.

**Claims**

1. A tuning method for a collimator of a CT machine, the CT machine comprising an anode plate (10), the collimator comprising a slot plate (20) and a controller (40), wherein the tuning method, in each cycle of an X-ray scan operation and a stop, comprises:

    a detection step (S10) of determining whether an X-ray is generated from the anode plate;
    if an X-ray is detected in the detection step (S10), the controller calculates a Z-direction heating displacement $\Delta z_{Thermal}^{(\alpha)}$ of the slot plate during the current scan (S20), wherein a indicates the current cycle;
    the controller drives the slot plate to move by the heating displacement in the Z-direction (S22);
    if no X-ray is detected in the detection step (S10), the controller calculates a total Z-direction heating displacement $z_{Thermal}^{(\alpha)}$ of the slot plate at the end of the current scan (S30);

    the controller, on the basis of the stop time of the X-ray, calculates a cooling displacement $z_{Thermal}^{(\alpha)\prime}$ of the slot plate during the current stop (S32); and the controller drives the slot plate to move (S34).

2. The tuning method according to claim 1, **characterized in that** the cooling displacement $z_{Thermal}^{(\alpha)\prime}$ at the time of the current stop is the difference between the total heating displacement $z_{Thermal}^{(\alpha)}$ of the current scan and $f(\Delta T_\alpha)$, a function of the current stop time $\Delta T_\alpha$, namely

$$z_{Thermal}^{(\alpha)'} = z_{Thermal}^{(\alpha)} - f(\Delta T_\alpha).$$

3. The tuning method according to claim 2, **characterized in that** $f(\Delta T_\alpha)$, the function of the current stop time, is a linear function, namely

$$f(\Delta T_\alpha) = \kappa \cdot \Delta T_\alpha,$$

wherein $\kappa$ is a thermal coefficient of the anode plate.

4. The tuning method according to any one of claims 1-3, **characterized in that** the tuning method, in each cycle of an X-ray scan operation and a stop, further comprises

comparing the cooling displacement $z_{Thermal}^{(\alpha)'}$ at the current stop with a predetermined cooling position $Z_{min}$ of the slot plate;

if the cooling displacement $z_{Thermal}^{(\alpha)'}$ is not smaller than the predetermined cooling position $Z_{min}$, the controller drives the slot plate to move by the cooling displacement $z_{Thermal}^{(\alpha)'}$ (S342);

if the cooling displacement $z_{Thermal}^{(\alpha)'}$ is smaller than the predetermined cooling position $Z_{min}$, the controller drives the slot plate to move by the predetermined cooling position $Z_{min}$ (S344).

5. The tuning method according to any one of claims 1-4, **characterized in that** the total heating displacement $z_{Thermal}^{(\alpha)}$ of the current scan is the sum of the total cooling displacement $z_{Thermal}^{(\alpha-1)'}$ of the previous scan and the heating displacement $\Delta z_{Thermal}^{(\alpha)}$ of the current scan, namely

$$z_{Thermal}^{(\alpha)} = z_{Thermal}^{(\alpha-1)'} + \Delta z_{Thermal}^{(\alpha)}.$$

6. The tuning method according to claim 5, **characterized in that** the heating displacement $\Delta z_{Thermal}^{(\alpha)}$ of the current scan is the sum of the displacements in successive movements made by the slot plate during the current scan, namely

$$\Delta z_{Thermal}^{(\alpha)} = \sum_{i=1}^{i=n} \Delta z_i^{(\alpha)},$$

wherein n is the total number of slot plate movements during the current scan.

7. The tuning method according to claim 5 or 6, **characterized in that** the heating displacement $\Delta z_{Thermal}^{(\alpha)}$ of the current scan is calculated by the controller on the basis of the thermal coefficient $\kappa_{H\alpha}$ of the current scan and the current scanning time $\Delta T_{H\alpha}$, namely

$$\Delta z_{Thermal}^{(\alpha)} = \kappa_{H\alpha} \cdot \Delta T_{H\alpha},$$

wherein $\kappa_{H\alpha}$ is determined by a lookup table stored in the controller.

8. The tuning method according to claim 7, **characterized in that** the tuning method, in each cycle of an X-ray scan operation and a stop, further comprises

comparing the calculated heating displacement $z_{Thermal}^{(\alpha)}$ of the current scan with a predetermined heating displacement $Z_{max}$ of the slot plate;

if the calculated heating displacement $z_{Thermal}^{(\alpha)}$ is not greater than the predetermined heating displacement $Z_{max}$, the controller drives the slot plate to move by the heating displacement $z_{Thermal}^{(\alpha)}$;

if the calculated heating displacement $z_{Thermal}^{(\alpha)}$ is greater than the predetermined heating displacement $Z_{max}$, the controller drives the slot plate to move by the predetermined heating displacement $Z_{max}$.

9. A controller for a collimator of a CT machine, the controller (40) comprising a control module for executing the steps as claimed any one of claims 1-8.

10. A collimator of a CT machine, comprising a slot plate (20) and a controller (40) as claimed in claim 9.

11. A CT machine, comprising a collimator as claimed in claim 10.

**Patentansprüche**

1. Abstimmverfahren für einen Kollimator einer CT-Maschine, wobei die CT-Maschine eine Anodenplatte (10) umfasst, wobei der Kollimator eine Schlitzplatte (20) und eine Steuerung (40) umfasst, wobei das Abstimmverfahren in jedem Zyklus einer Röntgenscanoperation und eines Stopps Folgendes umfasst:

einen Detektionsschritt (S 10) zum Bestimmen, ob ein Röntgenstrahl von der Anodenplatte erzeugt wird;
falls in dem Detektionsschritt (S 10) ein Röntgenstrahl detektiert wird, dann berechnet die Steuerung eine Z-Richtung-Heizungsverdrängung $\Delta z_{Thermisch}^{(\alpha)}$ der Schlitzplatte während des aktuellen Scans (S20), wobei $\alpha$ den aktuellen Zyklus angibt;
die Steuerung treibt die Schlitzplatte, um sich um die Heizungsverdrängung in Z-Richtung zu bewegen (S22);
falls in dem Detektionsschritt (S10) kein Röntgenstrahl detektiert wird, dann berechnet die Steuerung eine gesamte Z-Richtung-Heizungsverdrängung $z_{Thermisch}^{(\alpha)}$ der Schlitzplatte am Ende des aktuellen Scans (S30);
die Steuerung berechnet auf der Grundlage der Stoppzeit des Röntgenstrahls eine Kühlungsverdrängung $z_{Thermisch}^{(\alpha)\prime}$ der Schlitzplatte während des aktuellen Stopps (S32); und
die Steuerung treibt die Schlitzplatte, um sich zu bewegen (S34).

2. Abstimmverfahren nach Anspruch 1, **dadurch gekennzeichnet, dass** die Kühlungsverdrängung $z_{Thermisch}^{(\alpha)\prime}$ zum Zeitpunkt des aktuellen Stopps die Differenz zwischen der gesamten Heizungsverdrängung $z_{Thermisch}^{(\alpha)}$ des aktuellen Scans und $f(\Delta T_{\alpha})$ eine Funktion der aktuellen Stoppzeit $\Delta T_{\alpha}$ ist, nämlich

$$z_{Thermisch}^{(\alpha)\prime} = z_{Thermisch}^{(\alpha)} - f(\Delta T_{\alpha}).$$

3. Abstimmverfahren nach Anspruch 2, **dadurch gekennzeichnet, dass** $f(\Delta T_\alpha)$, die Funktion der aktuellen Stoppzeit, eine lineare Funktion ist, nämlich

$$f\ (\Delta T_\alpha)\ =\ \kappa \cdot \Delta T_\alpha,$$

wobei $\kappa$ ein Wärmekoeffizient der Anodenplatte ist.

4. Abstimmverfahren nach einem der Ansprüche 1 bis 3, **dadurch gekennzeichnet, dass** das Abstimmverfahren, in jedem Zyklus einer Röntgenscanoperation und eines Stopps, ferner Folgendes umfasst

Vergleichen der Kühlungsverdrängung $z^{(\alpha)\prime}_{Thermisch}$ an dem aktuellen Stopp mit einer vorbestimmten Kühlungsposition $Z_{min}$ der Schlitzplatte;

falls die Kühlungsverdrängung $z^{(\alpha)\prime}_{Thermisch}$ nicht kleiner als die vorbestimmte Kühlungsposition $Z_{min}$ ist, dann treibt die Steuerung die Schlitzplatte, um sich um die Kühlungsverdrängung zu bewegen $z^{(\alpha)\prime}_{Thermisch}$ (S342);

falls die Kühlungsverdrängung $z^{(\alpha)\prime}_{Thermisch}$ kleiner als die vorbestimmte Kühlungsposition $Z_{min}$ ist, dann treibt die Steuerung die Schlitzplatte, um sich um die vorbestimmte Kühlungsposition $Z_{min}$ (S344) zu bewegen.

5. Abstimmverfahren nach einem der Ansprüche 1 bis 4, **dadurch gekennzeichnet, dass** die gesamte Heizungsverdrängung $z^{(\alpha)}_{Thermisch}$ des aktuellen Scans die Summe der gesamten Kühlungsverdrängung $z^{(\alpha-1)\prime}_{Thermisch}$ des vorherigen Scans und der Heizungsverdrängung $\Delta z^{(\alpha)}_{Thermisch}$ des aktuellen Scans ist, nämlich

$$z^{(\alpha)}_{Thermisch} = z^{(\alpha-1)\prime}_{Thermisch} + \Delta z^{(\alpha)}_{Thermisch} \cdot$$

6. Abstimmverfahren nach Anspruch 5, **dadurch gekennzeichnet, dass** die Heizungsverdrängung $\Delta z^{(\alpha)}_{Thermisch}$ des aktuellen Scans die Summe der Verdrängungen in aufeinanderfolgenden Bewegungen ist, die von der Schlitzplatte während des aktuellen Scans gemacht werden, nämlich

$$\Delta z^{(\alpha)}_{Thermisch} = \sum_{i=1}^{i=n} \Delta z^{(\alpha)}_i,$$

wobei n die Gesamtanzahl von Schlitzplattenbewegungen während des aktuellen Scans ist.

7. Abstimmverfahren nach Anspruch 5 oder 6, **dadurch gekennzeichnet, dass** die Heizungsverdrängung $\Delta z^{(\alpha)}_{Thermisch}$ des aktuellen Scans von der Steuerung auf der Grundlage des thermischen Koeffizienten $\kappa_{H\alpha}$ des aktuellen Scans und der aktuellen Scanzeit $\Delta T_{H\alpha}$ berechnet wird, nämlich

$$\Delta z^{(\alpha)}_{Thermisch}\ =\ \kappa_{H\alpha} \cdot \Delta T_{H\alpha},$$

wobei $\kappa_{H\alpha}$ durch eine Nachschlagetabelle bestimmt wird, die in der Steuerung gespeichert ist.

8. Abstimmverfahren nach Anspruch 7, **dadurch gekennzeichnet, dass** das Abstimmverfahren in jedem Zyklus einer Röntgenscanoperation und eines Stopps ferner umfasst

Vergleichen der berechneten Heizungsverdrängung $z^{(\alpha)}_{Thermisch}$ des aktuellen Scans mit einer vorbestimmten

Heizungsverdrängung $Z_{max}$ der Schlitzplatte;

falls die berechnete Heizungsverdrängung $z_{Thermisch}^{(\alpha)}$ nicht größer als die vorbestimmte Heizungsverdrängung $Z_{max}$ ist, dann treibt die Steuerung die Schlitzplatte, um sich um die Heizungsverdrängung $z_{Thermisch}^{(\alpha)}$ zu bewegen;

falls die berechnete Heizungsverdrängung $z_{Thermisch}^{(\alpha)}$ größer als die vorbestimmte Heizungsverdrängung $Z_{max}$ ist, dann treibt die Steuerung die Schlitzplatte, um sich um die vorbestimmte Heizungsverdrängung $Z_{max}$ zu bewegen.

9. Steuerung für einen Kollimator einer CT-Maschine, wobei die Steuerung (40) ein Steuermodul zum Ausführen der Schritte nach einem der Ansprüche 1 bis 8 umfasst.

10. Kollimator einer CT-Maschine, der eine Schlitzplatte (20) und eine Steuerung (40) nach Anspruch 9 umfasst.

11. CT-Maschine, die einen Kollimator nach Anspruch 10 umfasst.


**Revendications**

1. Procédé de réglage pour un collimateur d'un appareil de tomodensitométrie, l'appareil de tomodensitométrie comprenant une plaque d'anode (10), le collimateur comprenant une plaque à fente (20) et un contrôleur (40), le procédé de réglage, dans chaque cycle d'une opération de balayage par rayons X et d'un arrêt, comprenant :

   une étape de détection (S10) visant à déterminer si un rayon X est généré à partir de la plaque d'anode ;
   si un rayon X est détecté à l'étape de détection (S10), le calcul, par le contrôleur, d'un décalage de chauffage dans la direction Z $\Delta z_{Thermal}^{(\alpha)}$ de la plaque à fente durant le balayage en cours (S20), $\alpha$ indiquant le cycle en cours ;
   l'entraînement, par le contrôleur, de la plaque à fente pour la déplacer du décalage de chauffage dans la direction Z (S22) ;
   si aucun rayon X n'est détecté à l'étape de détection (S10), le calcul, par le contrôleur, d'un décalage de chauffage total dans la direction Z $z_{Thermal}^{(\alpha)}$ de la plaque à fente à la fin du balayage en cours (S30) ;
   le calcul, par le contrôleur, sur la base du temps d'arrêt du rayon X, d'un décalage de refroidissement $z_{Thermal}^{(\alpha)\prime}$ de la plaque à fente durant l'arrêt en cours (S32) ; et
   l'entraînement, par le contrôleur, de la plaque à fente pour la déplacer (S34).

2. Procédé de réglage selon la revendication 1, **caractérisé en ce que** le décalage de refroidissement $z_{Thermal}^{(\alpha)\prime}$ au moment de l'arrêt en cours représente la différence entre le décalage de chauffage total $z_{Thermal}^{(\alpha)}$ du balayage en cours et $f(\Delta T_\alpha)$, une fonction du temps d'arrêt en cours $\Delta T_\alpha$, à savoir

$$z_{Thermal}^{(\alpha)\prime} = z_{Thermal}^{(\alpha)} - f(\Delta T_\alpha).$$

3. Procédé de réglage selon la revendication 2, **caractérisé en ce que** $f(\Delta T_\alpha)$, la fonction du temps d'arrêt en cours, est une fonction linéaire, à savoir

$$f(\Delta T_\alpha) = \kappa \cdot \Delta T_\alpha,$$

$\kappa$ étant un coefficient thermique de la plaque d'anode.

4. Procédé de réglage selon l'une quelconque des revendications 1 à 3, le procédé de réglage étant **caractérisé en ce**

**qu'**il comprend en outre, dans chaque cycle d'une opération de balayage par rayons X et d'un arrêt :

la comparaison du décalage de refroidissement $z_{Thermal}^{(\alpha)\prime}$ au moment de l'arrêt en cours à une position de refroidissement prédéterminée $Z_{min}$ de la plaque à fente ;

si le décalage de refroidissement $z_{Thermal}^{(\alpha)\prime}$ n'est pas inférieur à la position de refroidissement prédéterminée $Z_{min}$, l'entraînement, par le contrôleur, de la plaque à fente pour la déplacer du décalage de refroidissement $z_{Thermal}^{(\alpha)\prime}$ (S342) ;

si le décalage de refroidissement $z_{Thermal}^{(\alpha)\prime}$ est inférieur à la position de refroidissement prédéterminée $Z_{min}$, l'entraînement, par le contrôleur, de la plaque à fente pour la déplacer du décalage de refroidissement prédéterminé $Z_{min}$ (S344).

5. Procédé de réglage selon l'une quelconque des revendications 1 à 4, **caractérisé en ce que** le décalage de chauffage total $z_{Thermal}^{(\alpha)}$ du balayage en cours représente la somme du décalage de refroidissement total $z_{Thermal}^{(\alpha-1)\prime}$ du balayage précédent et du décalage de chauffage $\Delta z_{Thermal}^{(\alpha)}$ du balayage en cours, à savoir

$$z_{\text{Thermal}}^{(\alpha)} = z_{\text{Thermal}}^{(\alpha-1)\prime} + \Delta z_{\text{Thermal}}^{(\alpha)} \cdot$$

6. Procédé de réglage selon la revendication 5, **caractérisé en ce que** le décalage de chauffage $\Delta z_{Thermal}^{(\alpha)}$ du balayage en cours représente la somme des décalages lors de déplacements successifs effectués par la plaque à fente durant le balayage en cours, à savoir

$$\Delta z_{Thermal}^{(\alpha)} = \sum_{i=1}^{i=n} \Delta z_i^{(\alpha)},$$

n représentant le nombre total de déplacements de la plaque à fente durant le balayage en cours.

7. Procédé de réglage selon la revendication 5 ou 6, **caractérisé en ce que** le décalage de chauffage $\Delta z_{Thermal}^{(\alpha)}$ du balayage en cours est calculé par le contrôleur sur la base du coefficient thermique $\kappa_{H\alpha}$ du balayage en cours et du temps de balayage en cours $\Delta T_{H\alpha}$, à savoir

$$\Delta z_{Thermal}^{(\alpha)} = \kappa_{H\alpha} \cdot \Delta T_{H\alpha},$$

$\kappa_{H\alpha}$ étant déterminé à l'aide d'une table de correspondance stockée dans le contrôleur.

8. Procédé de réglage selon la revendication 7, le procédé de réglage étant **caractérisé en ce qu'**il comprend en outre, dans chaque cycle d'une opération de balayage par rayons X et d'un arrêt :

la comparaison du décalage de chauffage $z_{Thermal}^{(\alpha)}$ calculé du balayage en cours à un décalage de chauffage prédéterminé $Z_{max}$ de la plaque à fente ;

si le décalage de chauffage $z_{Thermal}^{(\alpha)}$ calculé n'est pas supérieur à la position de chauffage prédéterminée $Z_{max}$, l'entraînement, par le contrôleur, de la plaque à fente pour la déplacer du décalage de chauffage $z_{Thermal}^{(\alpha)}$ ;

si le décalage de chauffage $z^{(\alpha)}_{Thermal}$ calculé est supérieur à la position de chauffage prédéterminée $Z_{max}$, l'entraînement, par le contrôleur, de la plaque à fente pour la déplacer du décalage de chauffage prédéterminé $Z_{max}$.

9. Contrôleur pour un collimateur d'un appareil de tomodensitométrie, le contrôleur (40) comprenant un module de commande pour exécuter les étapes selon l'une quelconque des revendications 1 à 8.

10. Collimateur d'un appareil de tomodensitométrie, comprenant une plaque à fente (20) et un contrôleur (40) selon la revendication 9.

11. Appareil de tomodensitométrie, comprenant un collimateur selon la revendication 10.

**Z-direction** →

Fig. 1

Fig. 2

Start

$S10$

No

Yes

$\Delta Z^{(\alpha)}_{Thermal}$ — S20

$Z^{(\alpha)}_{Thermal}$ — S30

$\underline{S22}$

$Z^{(\alpha)'}_{Thermal}$ — S32

$\underline{S34}$

Fig. 3

$$Z^{(\alpha)'}_{Thermal} \leq Z_{min}$$

Fig. 4

X-ray scan or stop

Fig. 5

X-ray scan or stop

(a)

α-1    α    α+1    α+2

← Scan →    Scan →    ← Scan →

Stop →    ← Stop →

Time T

Z-direction
displacement

$\Delta T_1$    $\Delta T_2$    $\Delta T_3$    $\Delta T_4$    $\Delta T_5$

(b)

Time T

$Z_{Thermal}^{(\alpha-1)'}$    $Z_{Thermal}^{(\alpha)}$    $Z_{Thermal}^{(\alpha)'}$    $Z_{Thermal}^{(\alpha+1)}$    $Z_{Thermal}^{(\alpha+1)'}$    $z_{Thermal}^{(\alpha+2)}$

# Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8331527 B2